(19) Europäisches Patentamt
European Patent Office
Office européen des brevets

(11) Numéro de publication : **0 065 447**
**B1**

(12) **FASCICULE DE BREVET EUROPÉEN**

(45) Date de publication du fascicule du brevet : **05.09.84**

(21) Numéro de dépôt : **82400801.5**

(22) Date de dépôt : **03.05.82**

(51) Int. Cl.³ : **B 01 J 27/12**, B 01 J 21/08, B 01 J 35/10, C 07 C120/00, C 07 C121/00

(54) **Catalyseurs constitués de silice contenant du fluor, leur procédé de préparation et leur application à la préparation de nitriles.**

(30) Priorité : **15.05.81 FR 8109693**

(43) Date de publication de la demande : **24.11.82 Bulletin 82/47**

(45) Mention de la délivrance du brevet : **05.09.84 Bulletin 84/36**

(84) Etats contractants désignés : **AT BE CH DE FR GB IT LI LU NL SE**

(56) Documents cités :
**DE-C- 736 527**
**FR-A- 952 192**
**FR-A- 1 250 165**
**FR-A- 1 528 785**
**FR-A- 2 015 130**
**GB-A- 1 270 224**
**US-A- 2 477 695**
**US-A- 2 794 002**
**US-A- 3 514 478**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Jacques, Roland**
**579, Montée de silhol**
**F-30100 Alès (FR)**
Inventeur : **Reppelin, Michel**
**10, Chemin Neuf**
**F-69600 Collonges-au-Mont d'Or (FR)**
Inventeur : **Seigneurin, Laurent**
**3, avenue du Parc**
**F-30340 Salindres (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 065 447 B1

**Description**

La présente invention a pour objet des catalyseurs constitués de silice contenant du fluor. L'invention concerne également un procédé de préparation de telles silices et leur application à la préparation de nitriles aromatiques ou aliphatiques à partir des formanilides, formamides ou amides correspondants.

On connaît dans l'art antérieur, le brevet français 952.192 qui décrit, la préparation de silices contenant du fluor et du sodium à partir de silicate de sodium. Selon le procédé de préparation décrit, on ajoute à une solution d'acide sulfurique en excès, du silicate de sodium dissous et, ensuite, du fluorure de potassium. En d'autres termes, au cours de ce procédé, on assiste d'abord à la formation de silice par réaction de l'acide sulfurique sur le silicate de soude, puis l'excès d'acide sulfurique réagit avec le fluorure de potassium introduit ultérieurement pour donner de l'acide fluorhydrique qui imprègne alors le sol de silice formé. On obtient ainsi des silices contenant du fluor (le fluor est principalement sous forme de fluorosilicate de potassium) dont les surfaces spécifiques ne sont jamais inférieures à 450 $m^2/g$ mais au contraire souvent supérieures à 600 $m^2/g$ avec des volumes poreux au mieux voisins de 80 $cm^3/100$ g, le diamètre moyen des pores variant de 6 à 9 nm environ.

Selon le brevet précité, les silices obtenues sont utilisées comme catalyseurs, dans des procédés de cracking, reforming, isomérisation, polymérisation et alkylation.

La demanderesse a maintenant découvert de nouvelles silices fluorées à faible teneur en sodium dont les caractéristiques permettent leur application comme catalyseur dans une réaction, non mentionnée dans le brevet précité, à savoir la préparation de nitriles.

La présente invention a pour objet une silice contenant du fluor caractérisée en ce que sa surface spécifique est comprise entre 200 et 300 $m^2/g$, son volume poreux total est compris entre 1 et 1,5 $cm^3/g$, le diamètre moyen des pores est compris entre 10 et 20 nm, le pH d'échange est inférieur à 3 environ et en ce que la teneur en fluor lié à la silice exprimée en $F^-$ est comprise entre 0,05 et 2 % en poids par rapport à la silice, la teneur en sodium exprimée en $Na_2O$ étant inférieure à environ 1 % en poids par rapport à la silice.

On entend par pH d'échange, le pH mesuré pour une solution de 10 g de silice dans 100 g d'$H_2O$ pure à 20 °C.

On entend par fluor lié à la silice, le fluor non combiné sous les formes connues de fluosilicates ou de fluorures.

Selon un mode de réalisation préférentiel, les silices de l'invention ont une surface spécifique comprise entre 200 et 250 $m^2/g$, un volume poreux total compris entre 1 et 1,3 $cm^3/g$, un diamètre moyen des pores compris entre 10 et 25 nm, un pH d'échange compris entre 1 et 3, une teneur en fluor comprise entre 0,1 et 1 % et une teneur en sodium inférieure à 0,15 %.

Les silices fluorées selon l'invention se distinguent donc de celles de l'art antérieur en ce que ce sont des silices acides macroporeuses contenant du fluor lié directement à la silice.

L'invention concerne également un procédé de préparation de telles silices. Selon le trait fondamental du procédé de préparation découvert par la demanderesse qui le distingue nettement de l'art antérieur, la formation de la silice est obtenue par réaction du silicate de soude et de l'acide fluorhydrique.

En effet, l'invention a pour objet un procédé de préparation de silices fluorées contenant du sodium caractérisé en ce que l'on ajoute à une solution aqueuse d'acide fluorhydrique, une solution aqueuse de silicate de sodium, à une température comprise entre $-$ 5 °C et 15 °C tout en maintenant une teneur en $SiO_2$ inférieure à environ 15 % en poids du milieu réactionnel jusqu'à ce que le pH du milieu réactionnel atteigne une valeur comprise entre 3 et 4,5 ; l'on laisse gélifier le mélange ; l'on concasse en grains l'hydrogel obtenu ; l'on lave les grains obtenus pour éliminer les sels fluorés solubles et l'on sèche les grains lavés à une température comprise entre 150 et 600 °C.

Plus la température de mise en réaction du silicate de sodium et de l'acide fluorhydrique sera faible, plus le taux de $SiO_2$ pourra être élevé.

Plus le pH d'arrêt de la réaction sera faible, plus le temps de gélification ultérieure sera long.

Selon un mode de réalisation préférentiel, on utilise un silicate de sodium contenant du $SiO_2$ et du $Na_2O$ dans un rapport molaire égal à environ 3.

Selon un autre mode de réalisation préférentiel de l'invention, on met en œuvre entre 1 et 1,5 mole environ de HF par mole de $SiO_2$.

On maintient de préférence la température à une valeur comprise entre 0 et 10 °C pendant le mélange des solutions de silicate de sodium et d'acide fluorhydrique, la teneur en $SiO_2$ étant maintenue à une valeur inférieure à 10 %.

On arrête de préférence l'addition d'acide fluorhydrique lorsque le pH arrive à une valeur comprise entre 3,8 et 4,5.

Pour mettre en œuvre le procédé de l'invention, on peut utiliser une solution d'acide fluorhydrique très concentrées et une solution de silicate de sodium très diluée ou, au contraire, une solution d'acide fluorhydrique très diluée et une solution de silicate de soude très concentrée. Pour des raisons d'ordre pratique, on préfère utiliser les deux solutions à une concentration moyenne, par exemple voisine de 40 à 50 % en poids.

Pour une bonne mise en œuvre du procédé selon l'invention, on concasse l'hydrogel de façon à obtenir des grains de 2 à 6 mm de diamètre moyen.

Le lavage se fait par tous moyens connus de l'homme de l'art. Selon un mode de réalisation préférentiel, on utilise une eau de lavage ayant un pH compris entre 7 et 10 et on lave jusqu'à élimination complète des sels solubles, comme cela est bien connu de l'homme de l'art.

Le séchage final se fait de préférence entre 150 °C et 300 °C pendant 10 à 24 heures.

Selon une manière pratique, mais non obligatoire, de mettre en œuvre l'invention, on utilise simultanément à l'acide fluorhydrique, un acide carboxylique soluble en milieu aqueux comme l'acide acétique ce qui permet de mieux contrôler la variation de pH. De préférence, on utilise une quantité d'acide carboxylique telle que le rapport molaire de l'acide carboxylique à l'acide fluorhydrique est compris entre 0,05 et 0,15.

Un autre objet de l'invention est l'application des catalyseurs tels que définis ci-dessus à la préparation de nitriles aromatiques ou aliphatiques de formule :

$$Ar—A—CN \qquad (I)$$

dans laquelle :

— Ar représente un radical benzénique

— A représente un lien valentiel ou un radical hydrocarboné contenant de 1 à 6 atomes de carbone.

Selon l'invention, on porte à une température comprise entre 450 °C et 550 °C le formanilide ou formamide de formule :

$$Ar—A—NHCHO \qquad (II)$$

ou l'amide de formule :

$$Ar—A—CONH_2 \qquad (III)$$

où Ar et A ont la signification précédente, en présence d'une silice telle que définie précédemment.

On entend par radical benzénique, le radical phényle et les radicaux phényle comportant un ou plusieurs substituants. On peut citer comme exemples de tels substituants, les radicaux alkyle et alkoxy ayant de 1 à 6 atomes de carbone, les radicaux phényle et phénoxy, les radicaux F, $CF_3$, $OCF_3$, $SCF_3$, OH, Cl, Br, CN.

On peut citer non limitativement comme composés de formule I que l'on peut préparer selon le procédé de l'invention, les composés suivants : benzonitrile, trifluorométhyl-3 benzonitrile, trifluorométhyl-4 benzonitrile, méthoxy-4 benzonitrile, hydroxy-4 benzonitrile, fluoro-2 benzonitrile, fluoro-3 benzonitrile, fluoro-4 benzonitrile, chloro-2 benzonitrile, chloro-3 benzonitrile, chloro-4 benzonitrile, chloro-2 trifluorométhyl-5 benzonitrile, trifluorométhyl-3 chloro-4 benzonitrile, phénoxy-3 benzonitrile, bis-trifluorométhyl-3,5 benzonitrile, dichloro-2,6 benzonitrile, difluoro-2,4 benzonitrile, difluoro-2,6 benzonitrile, trifluorométhylthio-3 benzonitrile, trifluorométhoxy-4 benzonitrile, phénylacétonitrile, trifluorométhyl-3 phénylacétonitrile, trifluorométhyl-4 phénylacétonitrile, fluoro-4 phénylacétonitrile, chloro-4 phénylacétonitrile, fluoro-2 phénylacétonitrile, chloro-2 phénylacétonitrile, trifluorométhoxy-2 phénylacétonitrile, trifluorométhoxy-4 phénylacétonitrile, trifluorométhylthio-2 phénylacétonitrile, trifluorométhylthio-4 phénylacétonitrile, fluoro-2 méthyl-5 phénylacétonitrile, fluoro-3 méthyl-6 phénylacétonitrile, chloro-2 trifluorométhoxy-5 phénylacétonitrile, trifluorométhoxy-2 chloro-5 phénylacétonitrile, difluoro-2,5 phénylacétonitrile, difluoro-2,4 phénylacétonitrile.

On peut donner comme exemples non limitatifs de composés de formule II, les composés suivants : formanilide, trifluorométhyl-3 formanilide, trifluorométhyl-4 formanilide, méthoxy-4 formanilide, hydroxy-4 formanilide, fluoro-2 formanilide, fluoro-3 formanilide, fluoro-4 formanilide, chloro-2 formanilide, chloro-3 formanilide, chloro-4 formanilide, chloro-2 trifluorométhyl-5 formanilide, trifluorométhyl-3 chloro-4 formanilide, phénoxy-3 formanilide, bistrifluorométhyl-3,5 formanilide, dichloro-2,6 formanilide, difluoro-2,4 formanilide, difluoro-2,6 formanilide, trifluorométhylthio-3 formanilide, trifluorométhoxy-4 formanilide, benzylformamide, trifluorométhyl-3 benzylformamide, trifluorométhyl-4 benzylformamide, fluoro-4 benzylformamide, chloro-4 benzylformamide, fluoro-2 benzylformamide, chloro-2 benzylformamide, trifluorométhoxy-2 benzylformamide, trifluorométhoxy-4 benzylformamide, trifluorométhylthio-2 benzylformamide, trifluorométhylthio-4 benzylformamide, fluoro-2 méthyl-5 benzylformamide, fluoro-3 méthyl-6 benzylformamide, chloro-2 trifluorométhoxy-5 benzylformamide, trifluorométhoxy-2 chloro-5 benzylformamide, difluoro-2,5 benzylformamide, difluoro-2,4 benzylformamide.

Des exemples non limitatifs de composés III sont les suivants : trifluorométhyl-3 benzamide, trifluorométhyl-4 benzamide, fluoro-2 benzamide, fluoro-3 benzamide, fluoro-4 benzamide, trifluorométhyl-3 phénylacétamide, fluoro-4 phénylacétamide, trifluorométhoxy-4 phénylacétamide.

On connaît dans l'art antérieur, le brevet français 1.250.165 qui vise la production de nitriles à partir de composés N-formylaminés, par réaction de ces derniers à température élevée en phase gazeuse en présence, comme catalyseurs, d'acide silicique actif ou de silicates qui comportent au moins 75 % de pores ayant un rayon compris entre 10 et 200 A avec un rayon moyen des pores compris entre 20 et 100 A

et une surface de moins de 550 $m^2/g$ et qui contiennent un oxyde métallique comme l'oxyde de titane.

Les expériences de la demanderesse effectuées avec un catalyseur du type de celui décrit dans le brevet français 1.250.165 ont montré que lorsqu'on utilise comme composé de départ, un composé N-formylaminé comportant un substituant fluoré, on assiste à la formation de produits secondaires lourds, ce qui diminue la durée de vie du catalyseur par encrassement de la surface et on assiste à la formation de composés résultant de la défluoration du radical fluoré, composés très difficilement séparables du produit recherché.

La demanderesse a constaté que, d'une façon surprenante, la mise en œuvre d'un catalyseur selon l'invention permet de diminuer très notablement la formation de produits lourds et d'éliminer pratiquement la défluoration du radical fluoré. Ceci est d'un intérêt industriel important car les nitriles obtenus selon le procédé de l'invention sont de très utiles intermédiaires de synthèse pour la préparation de composés à activité phytosanitaire et pharmaceutique.

Il en découle que le procédé selon l'invention est plus particulièrement, mais non exclusivement, adapté à la mise en œuvre des composés de formule II ou III dont le radical phényle comporte un ou plusieurs substituants fluorés F, $CF_3$, $OCF_3$ ou $SCF_3$.

Le procédé est encore plus adapté à la mise en œuvre de composés de formule II comportant un radical fluoré.

Parmi ces derniers, on peut citer le métatrifluorométhylformanilide :

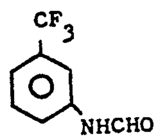

et le métatrifluorométhyl benzylformamide de formule :

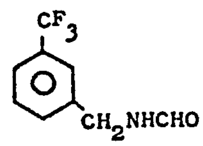

Selon une manière avantageuse, mais non obligatoire, de mettre en œuvre l'invention, on opère en présence d'un diluant gazeux inerte constitué de préférence par l'azote et/ou le $CO_2$ et/ou l'acétonitrile.

On préfère utiliser l'acétonitrile en quantité telle que le pourcentage molaire de composé de formule II ou III dans l'acétonitrile soit compris entre 2 et 20 et de préférence entre 5 et 10.

La température sera, préférentiellement comprise entre environ 510 et 530 °C quand on mettra en œuvre un formamide ou un formanilide entre 450 et 480 °C quand on mettra en œuvre un amide.

On opère généralement à la pression atmosphérique bien que des pressions supérieures ou inférieures à la pression atmosphérique ne soient pas exclues du domaine de l'invention.

On met en œuvre le procédé selon l'invention en faisant passer de préférence entre 0,2 et 4 moles de composé II ou III par heure et par litre de catalyseur.

Les composés III peuvent être préparés par toute technique connue de l'homme de l'art.

La préparation des composés II quand A est un lien valentiel se fait, d'une façon bien connue dans l'art antérieur, par réaction de l'aniline correspondante avec l'acide formique.

Dans le cas où A est un radical hydrocarboné comme $—CH_2—$ les composés II peuvent être obtenus par réaction à 0-100 °C en présence d'acide fluorhydrique entre le dérivé benzénique correspondant ArH et l'hydroxyméthylformamide $OH—CH_2—NHCHO$. Le rapport de l'acide fluorhydrique à ArH est compris entre environ 5 et 50 et celui de ArH à $OH—CH_2—NHCHO$ est compris entre environ 0,5 et environ 2. Cette préparation fait l'objet d'une demande de brevet au nom de la demanderesse.

D'autres caractéristiques et avantages de la présente invention apparaitront plus clairement à la lecture des exemples qui vont suivre. Ces exemples ne sauraient en aucune manière être considérés comme une limitation de l'invention.

## Exemple 1

On prépare une solution contenant 250 g d'HF à 40 %, 34,7 g d'acide acétique à 99 % et 712 g d'eau. On prépare une deuxième solution de silicate de soude de densité 1,185 ($SiO_2/Na_2O = 3,3$) ; Ces deux solutions sont refroidies à 5 °C. La solution de silicate est versée dans la solution d'acide fluorhydrique vigoureusement agitée tout en maintenant la température à 5 °C jusqu'à ce que le pH atteigne 4. On a alors versé 2 133 g de solution de silicate de soude. Le sol obtenu gélifie en 10 minutes. L'hydrogel obtenu est concassé de façon à obtenir des grains ayant un diamètre moyen de 2 à 6 mm. Ces grains sont lavés en colonne par un courant d'eau dont le pH a été amené à 8 par addition d'ammoniaque à température ambiante pendant 24 heures. Les solides obtenus sont ensuite séchés à 200 °C pendant

24 heures. Les caracteristiques de la silice obtenue sont les suivantes :

Surface spécifique : 223 $m^2/g$
Volume poreux total : 1,30 $cm^3/g$
Diamètre moyen des pores : 12 nm
Teneur en sodium ($Na_2O$) : 0,072 %
Teneur en fluor : 0,32 %
pH d'échange : 3.

Exemple 2

On prépare une solution contenant 250 g d'HF à 40 %, 34,7 g d'acide acétique à 99 % et 712 g d'$H_2O$. On prépare une deuxième solution de silicate de soude de densité 1,185 ($SiO_2/Na_2O$ = 3,3). Ces deux solutions sont refroidies à 10 °C. La solution de silicate est versée dans la solution d'acide fluorhydrique vigoureusement agitée jusqu'à ce que le pH atteigne 4. On a alors versé 1 720 g de solution de silicate de soude tout en maintenant la température à 10 °C. On verse la solution résultante sur une plaque où elle gélifie en 10 mn. L'hydrogel est concassé en grains de 2 à 6 mm de diamètre moyen qui sont lavés avec de l'eau ayant un pH de 8. Les solides obtenus sont séchés à 150 °C pendant 24 heures, relavés à l'eau distillée 36 heures puis séchés à 200 °C pendant 24 heures. Les caractéristiques de la silice obtenue sont :

Surface spécifique : 211 $m^2/g$
Volume poreux total : 1,03 $cm^3/g$
Diamètre moyen des pores : 11,9 nm
Teneur en sodium ($Na_2O$) : 0,046 %
Teneur en fluor : 0,26 %
pH d'échange : 3.

Exemple 3

On prépare une solution contenant 500 g d'HF à 40 % et 2 470 g d'eau puis une deuxième solution de silicate de soude de densité 1,185 ($SiO_2 - Na_2O$ = 3,3).

Elles sont toutes les deux refroidies à 0 °C.

La solution de silicate est versée dans la solution d'acide fluorhydrique vigoureusement agitée tout en maintenant la température entre entre − 2 et 2 °C jusqu'à ce que le pH atteigne 4 (la concentration de la silice dans le sol est de 10,67 %).

On a alors versé 2 097 g de solution de silicate de soude. Le sel obtenu gélifie en 20 minutes. L'hydrogel est concassé de façon à obtenir des grains ayant un diamètre moyen de 2 à 6 mm. Ces grains sont plongés dans 5 fois leur volume d'eau pendant une heure, le pH de l'eau ayant été amené à 8 par introduction d'ammoniaque.

Les solides obtenus sont ensuite séchés à 200 °C pendant 24 heures.

La silice est lavée par de l'eau déminéralisée puis séchée. Les caractéristiques de la silice sont les suivantes :

Surface spécifique : 233 $m^2/g$
Volume poreux total : 115 $cm^3/100$ g
Diamètre moyen des pores : 11,5 nm
Teneur en $Na_2O$ : 1 200 ppm
Teneur en fluor : 0,16 %
pH d'échange : 3.

Exemple 4

On prépare une solution contenant 500 g d'HF à 40 % et 5 000 $cm^3$ d'$H_2O$ puis une deuxième solution de silicate de soude de densité 1,185 ($SiO_2 - Na_2O$ = 3,3).

Elles sont toutes les deux refroidies à 10 °C.

La solution de silicate est versée dans la solution d'acide fluorhydrique vigoureusement agitée jusqu'à ce que le pH atteigne 4.

Il a été alors versé 2 370 g de solution de silicate de Na tout en maintenant la température entre 8 et 12 °C. La concentration en $SiO_2$ dans le sol est 6,77 %. On verse la solution résultante sur une plaque où elle gélifie en 10 minutes. L'hydrogel est concassé en grains de 2 à 6 mm de diamètre moyen. Ces grains sont plongés dans cinq fois leur volume d'eau, pendant 1 heure, le pH ayant été amené à 8 par introduction d'ammoniaque.

Les solides obtenus sont ensuite séchés à 200 °C pendant 24 heures. La silice séchée est lavée par de l'eau déminéralisée puis séchée.

Les caractéristiques de la silice obtenue sont les suivantes :

Surface spécifique : 195 $m^2/g$
Volume poreux total : 103 $cm^3/100$ g
Diamètre des pores : 12 nm
Teneur en $Na_2O$ : 1 600 ppm
Teneur en fluor : 0,31 %
pH d'échange : 3.

Exemple 5

Dans un réacteur tubulaire en acier inoxydable d'un litre et rempli de catalyseur tel que préparé à l'exemple 2, on introduit au départ en continu un mélange de 500 g/h d'acétonitrile et 125 g/h de métatrifluorométhylformanilide, préalablement préchauffé. La température réactionnelle est maintenue à 530 °C tout au long du lit catalytique. On obtient ainsi une conversion de 95 % du formanilide. Au cours du temps, les débits sont modifiés de façon à maintenir ce taux de transformation. On introduit ainsi en 450 heures, 37,20 kg de métatrifluorométhylformanilide. On récupère après distillation du solvant et de l'eau formée :

25,0 kg de métatrifluorométhyl benzonitrile
5,68 kg de métatrifluorométhyl aniline
2,39 kg du formanilide non transformé
0,52 kg de produits lourds.

La sélectivité en nitrile formé sur le formanilide réagi est de 98,1 %.

On définit dans cet exemple et dans les exemples ultérieurs, la sélectivité comme étant le rapport du nitrile formé au formanilide ayant réagi à l'exclusion du formanilide transformé en aniline correspondante puisque cette dernière, dans un procédé industriel, peut être transformée quantitativement par l'acide formique en formanilide de départ alors recyclé. La teneur en fluorures dans le mélange brut sortant du réacteur est de 130 ppm. Le métatrifluorométhylbenzonitrile ne contient que des traces de benzonitrile et seulement 0,32 % de métatolunitrile.

Exemple 6

En opérant comme dans l'exemple 5 et en utilisant la silice de l'exemple 1, on introduit en 390 heures, 44,46 kg de métatrifluorométhylformanilide. On récupère après distillation du solvant et de l'eau formée :

28,81 kg de mététrifluorométhyl benzonitrile
9,8 kg de métatrifluorométhylaniline
2,05 kg de métatrifluorométhylformanilide non transformé
1,26 kg de produits lourds.

La sélectivité en nitrile formé est de 95,9 %. La teneur en fluorures dans le mélange brut est de 100 ppm. Le nitrile obtenu après distillation ne contient que des traces de benzonitrile et 0,28 % de métatolunitrile.

Essai comparatif : On opère comme ci-dessus mais en remplaçant le catalyseur par un catalyseur au $TiO_2$ obtenu selon le procédé décrit dans l'exemple 1 du brevet français 1.250.165 précité. On introduit en 135 heures, 14,17 kg de métatrifluorométhylformanilide. On obtient :

9,93 kg de métatrifluorométhyl benzonitrile
0,85 kg de métatrifluorométhyl aniline
0,70 kg de métatrifluorométhyl formanilide non transformé
1,5 kg de produits lourds.

La sélectivité en nitrile formé est de 88 %. La teneur en fluorures dans le milieu réactionnel est de 1 630 ppm. Le nitrile obtenu contient 0,35 % de benzonitrile et 1,45 % de métatolunitrile. Au bout de 135 heures, le catalyseur est désactivé.

Exemple 7

On opère comme dans l'exemple 5 mais avec une température de la zone catalytique fixée à 510 °C et en alimentant le réacteur avec 600 g/h d'acétonitrile et 150 g/h de parafluoroformanilide. En 500 heures, 75 kg de parafluoroformanilide sont introduits et sont récupérés, après distillation du solvant :

55 kg de parafluorobenzonitrile
7,1 kg de parafluoroaniline
0,75 kg de parafluoroformanilide non transformé
3,02 kg de produits lourds.

La sélectivité en nitrile est de 96,7 %. La teneur en fluorures dans le mélange réactionnel brut est inférieure à 50 ppm. Au bout de 500 heures, le catalyseur est encore actif.

Exemple 8

On opère comme dans l'exemple 5 avec une température de la zone catalytique fixée à 520 °C et en alimentant le réacteur avec 48,36 kg de métatrifluorométhylbenzylformamide et 193,4 kg d'acétonitrile pendant 450 heures. En fin de réaction, après distillation du solvant et de l'eau formée, on récupère :

38,7 kg de métatrifluorométhylphénylacétonitrile
3,05 kg de métatrifluorométhylbenzylamine
1,5 kg de métatrifluorométhylbenzylformamide non transformé
0,9 kg de produits lourds.

La sélectivité en nitrile est de 98 %. La teneur en fluorures dans le mélange brut réactionnel est de 160 ppm. Le nitrile obtenu après distillation contient moins de 0,10 % de phénylacétonitrile et 0,4 % de métaméthylphénylacétonitrile.

Exemple 9

Dans le même appareillage que celui décrit à l'exemple 3 et avec le catalyseur de l'exemple 2, on introduit en 400 heures, un mélange de 45 kg de paratrifluorométhoxyformanilide et de 180 kg d'acétonitrile, la zone réactionnelle étant maintenue à 520 °C. On obtient :

35,2 kg de paratrifluorométhoxy benzonitrile
2,9 kg de paratrifluorométhoxy aniline
1,82 kg de paratrifluorométhoxyformanilide non transformé
1,26 kg de produits lourds.

La sélectivité en nitrile est de 96,9 %. La teneur en fluorures dans le milieu réactionnel est de 130 ppm.

Exemple 10

On opère comme dans l'exemple 5 en remplaçant une partie de l'acétonitrile par du gaz carbonique, c'est-à-dire en introduisant pendant les 100 premières heures sur la masse catalytique, 350 g/h d'acétonitrile, 125 g/h de métatrifluorométhylformanilide et 72 l/h TPN de gaz carbonique. On introduit ainsi en 450 heures, la même quantité de formanilide que dans l'exemple 2. Les résultats obtenus sont identiques à ceux obtenus dans l'exemple 3.

Exemple 11

En opérant comme dans l'exemple 6, avec le catalyseur obtenu selon l'exemple 3 avec une température du lit catalytique de 450 °C, on introduit un mélange de 125 g/h de métatrifluorométhylbenzamide et 500 g/h d'acétonitrile. La transformation est totale. On recueille en sortie de réacteur après condensation, un mélange de métatrifluorométhylbenzonitrile, acétonitrile et eau, et des traces de produits lourds. La teneur en fluorures dans le mélange réactionnel est inférieur à 50 ppm. La sélectivité en trifluorométhylbenzonitrile est supérieure à 99 %. Après 500 heures, le catalyseur est toujours actif.

Exemple 12

En opérant comme dans l'exemple 5, avec le catalyseur obtenu selon l'exemple 4, on introduit en continu, un mélange de 31 kg de paraméthoxyformanilide et de 144 kg d'acétonitrile. Après 400 heures de marche, on a recueilli à la sortie :

22,6 kg de paraméthoxybenzonitrile
2,61 kg de paraméthoxy aniline
1,24 kg du formanilide non transformé
0,88 kg de produits lourds.

La sélectivité en nitrile est de 96,7 %. Au bout de 400 heures, le catalyseur est toujours actif.

**Revendications**

1. Silice contenant du fluor caractérisée en ce que sa surface spécifique est comprise entre 200 et 300 $m^2/g$, son volume poreux total est compris entre 1 et 1,5 $cm^3/g$, le diamètre moyen des pores est compris entre 10 et 20 nm, le pH d'échange est inférieur à 3 environ et en ce que la teneur en fluor exprimée en $F^-$ est comprise entre 0,05 et 2 % en poids par rapport à la silice, la teneur en sodium exprimée en $Na_2O$ étant inférieure à 1 % environ en poids par rapport à la silice.

2. Silice selon la revendication 1 caractérisée en ce que la surface spécifique est comprise entre 200 et 250 $m^2/g$, le volume poreux total est compris entre 1 et 1,3 $cm^3/g$, le diamètre moyen des pores est compris entre 10 et 15 nm, le pH d'échange est compris entre 1 et 3, la teneur en fluor est comprise entre 0,1 et 1 % et la teneur en sodium est inférieure à 0,15 %.

3. Procédé de préparation d'une silice selon l'une quelconque des revendications précédentes caractérisé en ce que l'on ajoute à une solution aqueuse d'acide fluorhydrique, une solution aqueuse de silicate de soude à une température comprise entre − 5 °C et 15 °C tout en maintenant une teneur en $SiO_2$ inférieure à environ 15 % en poids du milieu réactionnel jusqu'à ce que le pH du milieu réactionnel atteigne une valeur comprise entre 3 et 4,5 ; l'on laisse gélifier le mélange ; l'on concasse en grains l'hydrogel obtenu ; l'on lave les grains obtenus pour éliminer les sels fluorés solubles et l'on sèche les grains lavés à une température comprise entre 150 °C et 600 °C.

4. Procédé selon la revendication 3 caractérisé en ce que le silicate de soude contient du $SiO_2$ et du $Na_2O$ dans un rapport molaire égal à environ 3.

5. Procédé selon la revendication 3 caractérisé en ce que l'on met en œuvre entre 1 et 1,5 mole de HF en mole de $SiO_2$.

6. Procédé selon la revendication 3 caractérisé en ce que l'on met en œuvre simultanément à l'acide fluorhydrique un acide carboxylique soluble en milieu aqueux.

7. Procédé selon la revendication 6 caractérisé en ce que l'acide carboxylique est utilisé en quantité telle que le rapport molaire de l'acide carboxylique à l'acide fluorhydrique est compris entre 0,05 et 0,15.

8. Procédé de préparation de nitriles aromatiques ou aliphatique de formule :

$$Ar—A—CN \quad (I)$$

dans laquelle :

— Ar représente un radical benzénique

— A représente un lien valentiel ou un radical hydrocarboné contenant de 1 à 6 atomes de carbone caractérisé en ce que l'on porte à une température comprise entre environ 450 °C et environ 550 °C le formamide ou formanilide de formule :

$$Ar—A—NHCHO \quad (II)$$

ou l'amide de formule :

$$Ar—A—CONH_2 \quad (III)$$

où Ar et A ont la signification précédente en présence d'une silice selon la revendication 1 ou 2.

9. Procédé selon la revendication 8 caractérisé en ce que dans les formules II et III, Ar est un radical phényl substitué par au moins un radical choisi parmi le groupe comprenant F, $CF_3$, $OCF_3$, $SCF_3$.

10. Procédé selon la revendication 9 caractérisé en ce que Ar représente le radical trifluorométhylphényle.

11. Procédé selon l'une quelconque des revendications 8 à 10 caractérisé en ce que l'on opère en outre, en présence d'au moins un diluant choisi parmi le groupe comprenant l'azote, le $CO_2$ et l'acétonitrile.

12. Procédé selon la revendication 11 caractérisé en ce que le diluant est l'acétonitrile.

**Claims**

1. Silica containing fluorine, characterised in that its specific surface area is between 200 and 300 $m^2/g$, its total pore volume is between 1 and 1.5 $cm^3/g$, the mean diameter of the pores is between 10 and 20 nm, the exchange pH is less than about 3 and the fluorine content expressed as $F^-$ is between 0.05 and 2 % by weight relative to the silica, the sodium content expressed as $Na_2O$ being less than about 1 % by weight relative to the silica.

2. Silica according to Claim 1, characterised in that the specific surface area is between 200 and 250 $m^2/g$, the total pore volume is between 1 and 1.3 $cm^3/g$, the mean diameter of the pores is between 10 and 15 nm, the exchange pH is between 1 and 3, the fluorine content is between 0.1 and 1 % and the sodium content is less than 0.15 %.

3. Process for the preparation of a silica according to either of the preceding claims, characterised in

that an aqueous sodium silicate solution is added to an aqueous hydrofluoric acid solution at a temperature of between − 5 °C and 15 °C whilst maintaining an $SiO_2$ content of less than about 15 % by weight of the reaction mixture, until the pH of the reaction mixture reaches a value of between 3 and 4.5, the mixture is allowed to gel, the hydrogel obtained is crushed into grains, the grains obtained are washed to remove the soluble fluorine-containing salts and the washed grains are dried at a temperature of between 150 °C and 600 °C.

4. Process according to Claim 3, characterised in that the sodium silicate contains $SiO_2$ and $Na_2O$ in a molar ratio of about 3.

5. Process according to Claim 3, characterised in that between 1 and 1.5 moles of HF are employed per mole of $SiO_2$.

6. Process according to Claim 3, characterised in that a carboxylic acid soluble in an aqueous medium is employed simultaneously with hydrofluoric acid.

7. Process according to Claim 6, characterised in that the carboxylic acid is used in such amount that the molar ratio of carboxylic acid to hydrofluoric acid is between 0.05 and 0.15.

8. Process for the preparation of aromatic or aliphatic nitriles of the formula :

$$Ar—A—CN \quad (I)$$

in which :

— Ar represents a benzene radical and

— A represents a valency bond or a hydrocarbon radical containing from 1 to 6 carbon atoms,

characterised in that the formamide or formanilide of the formula :

$$Ar—A—NHCHO \quad (II)$$

or the amide of the formula :

$$Ar—A—COHN_2 \quad (III)$$

where Ar and A have the above meaning is heated to a temperature of between about 450 °C and about 550 °C in the presence of a silica according to Claim 1 or 2.

9. Process according to Claim 8, characterised in that in the formulae II and III, Ar is a phenyl radical substituted by at least one radical chosen from amongst the group comprising F, $CF_3$, $OCF_3$ and $SCF_3$.

10. Process according to Claim 9, characterised in that Ar represents the trifluoromethylphenyl radical.

11. Process according to any one of Claims 8 to 10, characterised in that it is moreover carried out in the presence of at least one diluent chosen from amongst the group comprising nitrogen, $CO_2$ and acetonitrile.

12. Process according to Claim 11, characterised in that the diluent is acetonitrile.

**Ansprüche**

1. Fluorhaltiges Siliziumdioxid, dadurch gekennzeichnet, daß seine spezifische Oberfläche zwischen 200 und 300 $m^2/g$, sein gesamtes Porenvolumen zwischen 1 und 1,5 $cm^3/g$, der mittlere Porendurchmesser zwischen 10 und 20 nm, der Austausch-pH-Wert unter ca. 3, der Fluorgehalt, ausgedrückt als $F^-$, zwischen 0,05 und 2 Gew.-%, bezogen auf das Siliziumdioxid und der Natriumgehalt, ausgedrückt in $Na_2O$, unter ca. 1 Gew.-%, bezogen auf das Siliziumdioxid, liegen.

2. Siliziumdioxid nach Anspruch 1, dadurch gekennzeichnet, daß die spezifische Oberfläche zwischen 200 und 250 $m^2/g$, das gesamte Porenvolumen zwischen 1 und 1,3 $cm^3/g$, der mittlere Porendurchmesser zwischen 10 und 15 nm, der Austausch-pH-Wert zwischen 1 und 3, der Fluorgehalt zwischen 0,1 und 1 % und der Natriumgehalt unter 0,15 % liegen.

3. Verfahren zur Herstellung eines Siliziumdioxids nach Anspruch 1 oder 2, dadurch gekennzeichnet, daß man einer wässrigen Lösung aus Fluorwasserstoffsäure eine wässrige Natriumsilikat-Lösung bei einer Temperatur zwischen − 5°C und 15 °C zusetzt, wobei der $SiO_2$-Gehalt im Reaktionsgemisch bei einem Wert unter ca. 15 Gew.-% gehalten wird, bis der pH-Wert des Reaktionsgemisches einen Wert von 3 bis 4,5 erreicht, daß man das Gemisch gelieren läßt, das erhaltene Hydrogel zu Körnern zerkleinert, die erhaltenen Körner zur Entfernung der wasserlöslichen Fluorsalze wäscht und die gewaschenen Körner bei einer Temperatur zwischen 150 °C und 600 °C trocknet.

4. Verfahren nach Anspruch 3, dadurch gekennzeichnet, daß das Natriumsilikat $SiO_2$ und $Na_2O$ in einem Molverhältnis von ca. 3 enthält.

5. Verfahren nach Anspruch 3, dadurch gekennzeichnet, daß man zwischen 1 und 1,5 Mol HF pro Mol $SiO_2$ einsetzt.

6. Verfahren nach Anspruch 3, dadurch gekennzeichnet, daß man gleichzeitig mit der Fluorwasserstoffsäure eine in wässrigem Milieu lösliche Carbonsäure einsetzt.

7. Verfahren nach Anspruch 6, dadurch gekennzeichnet, daß die Carbonsäure in einer solchen Menge verwendet wird, daß das Molverhältnis der Carbonsäure zur Fluorwasserstoffsäure zwischen 0,05 und 0,15 liegt.

8. Verfahren zur Herstellung von aromatischen oder aliphatischen Nitrilen der Formel :

$$Ar—A—CN \qquad (I)$$

in der :

— Ar einen Benzolrest

— A eine Valenzbindung oder einen Kohlenwasserstoffrest mit 1 bis 6 Kohlenstoffatomen bedeuten, dadurch gekennzeichnet, daß man das Formamid oder Formanilid der Formel :

$$Ar—A—NHCHO \qquad (II)$$

oder das Amid der Formel :

$$Ar—A—CONH_2 \qquad (III)$$

wobei Ar und A die obige Bedeutung haben, in Anwesenheit von Siliziumdioxid nach Anspruch 1 oder 2 auf eine Temperatur zwischen etwa 450 °C und etwa 550 °C bringt.

9. Verfahren nach Anspruch 8, dadurch gekennzeichnet, daß in den Formeln II und III Ar ein Phenylrest ist, der durch mindestens einen der Reste F, $CF_3$, $OCF_3$, $SCF_3$ substituiert ist.

10. Verfahren nach Anspruch 9, dadurch gekennzeichnet, daß Ar der Trifluoromethylphenylrest ist.

11. Verfahren nach einem der Ansprüche 8 bis 10, dadurch gekennzeichnet, daß man außerdem in Anwesenheit mindestens eines Verdünnungsmittels aus der Gruppe von Stickstoff, $CO_2$ und Acetonitril arbeitet.

12. Verfahren nach Anspruch 11, dadurch gekennzeichnet, daß das Verdünnungsmittel Acetonitril ist.